# EUROPEAN PATENT SPECIFICATION

(11) **EP 3 741 350 B1**
(45) Date of publication and mention of the grant of the patent: **02.08.2023**
(21) Application number: 20175499.1
(22) Date of filing: 19.05.2020
(51) Int. Cl.: A61J 15/00

(54) **CONNECTOR FOR A GASTROSTOMY DEVICE**
VERBINDER FÜR EINE GASTROSTOMIEVORRICHTUNG
CONNECTEUR POUR UN DISPOSITIF DE GASTROSTOMIE

(30) Priority: 20.05.2019 GB 201907070
(43) Date of publication of application: 25.11.2020
(73) Proprietor: GBUK Group Limited, Selby, North Yorkshire YO8 5DD (GB)
(72) Inventor: HEYNES, Mark David, Newcastle-under-Lyme, Staffordshire ST5 0HY (GB); SAMPEY, Phil Anthony, Alsager, Cheshire ST7 2FE (GB); MORRIS, William Charles, Newcastle-under-Lyme, Staffordshire ST5 0LE (GB); SMITH, Lee Thomas, Stafford, Staffordshire ST18 0XU (GB)
(74) Representative: Murgitroyd & Company

(56) References cited:
- JP-A- 2005 058 638
- US-A1- 2012 029 483
- US-A1- 2014 155 839
- US-A1- 2014 155 866

## Description

### Field of the invention

The invention relates to a connector for connection of an enteral feeding solution supply tube to a gastrostomy device, and non-surgical and non-therapeutic methods of use thereof. Such a device is disclosed in the US2012029483.

### Background to the invention

Many patients are incapable of taking nourishment in the conventional fashion. This might be as a result of a pathology or surgery. The process of feeding a patient by the use of an enteral feeding device is well known in the art. Typically, a gastrostomy device is implanted in a patient's stomach wall. The gastrostomy device is then connected via a connector or adapter to an enteral feeding tube. Nutritional fluids are then transported via the gastrostomy device into the patient's stomach.

One of the common problems associated with this process is that the feeding tube and adapter may become accidentally disengaged from the gastrostomy device during the course of feeding, and thus the flow of nutritional fluids to the patient is interrupted.

There is a need for an improved connector for retaining a feeding tube and gastrostomy device in an interlocked position relative to one another.

### Summary of the invention

According to a first aspect of the invention there is provided a connector as further disclosed in the claims, for rotatably coupling an enteral feeding solution supply tube to a circular port of a gastrostomy device, the connector comprising:
a fluid conduit having a first end configured for connection with an orifice defined in the circular port to supply the enteral feeding solution to the gastrostomy device, and a second end configured for connection to the enteral feeding solution supply tube,
a pair of arms circumferentially arranged around an outer wall of the first end of the fluid conduit, each arm including a first free end and a second free end, wherein the second free end includes a catch configured to releasably engage with a circumferential rim on the circular port, and
a flexible bridge connecting each arm to the first end of the fluid conduit and about which the first and second ends of each arm can be pivoted,
wherein the connector is couplable to and decouplable from the circular port by pivoting the first and second ends of each arm at the flexible bridge such that the catch on the second free end of each arm is radially displaced into engagement or out of engagement with the rim.

The design of the connector enables the connector to be securely connected to circular port of the gastrostomy device, whilst also allowing rotation of the connector about the port. The gastrostomy device is often referred to in the art as a "button". The circular port is often referred to in the art as an "interlock".

The outer wall of the first end of the fluid conduit and an inner wall of each arm are substantially parallel. This allows the arm to pivot and reduces the distance that the arm needs to travel during engagement and disengagement from the rim of the interlock. Accordingly, this puts less stress on the material from which the arm is formed, and minimises the risk of breakage.

The catch is located on the inner wall of the arm. The catch may be defined by a ledge that projects inwardly towards the first end of the fluid conduit. The ledge may have a rim contacting surface configured to releasably engage with a ledge contacting surface of the circumferential rim.

The ledge contacting surface of the rim is a sloped surface, and the rim contacting surface of the ledge has a complementary sloped surface. This design improves the retention of the catch against the underside of the ledge contacting surface of the circumferential rim by creating a hook effect.

It is desirable that the connector is not disengaged from the gastrostomy device during the feeding process. Accordingly, the connector as defined herein may further comprise a locking member at least a part of which is configured to be sandwiched between the outer wall of the fluid conduit and the inner wall of each arm to prevent inadvertent pivoting of each arm when the connector is coupled to the port of the gastrostomy device.

In some constructions, the lock member comprises a pair of hooks configured to hook around at least part of each flexible bridge. For example, each hook may be configured to hook underneath each flexible bridge.

The lock member may further comprise a hinge about which the hooks can be pivoted into and out of engagement with each flexible bridge. For example, in some constructions wherein the second end of the fluid conduit is substantially perpendicular to the first end of the fluid conduit, wherein the lock member comprises:
a collar for connecting the lock member to the second end of the fluid conduit, and
a cover portion hingedly attached to the collar, wherein a pair of hooks extend from the cover portion,
and wherein the cover portion is biased such that the surface from which the hooks depend is substantially perpendicular to the second end of the fluid conduit.

The lock member may be removably attachable to the connector. For example, the collar of the lock member may comprise a closed ring that has an inner diameter that is slightly larger than the outer diameter of the second end of the connector. This enables the collar of the lock member to be slidably attached to the second end of the connector. In some other constructions, the collar of the lock member may comprise an open ring that is made of a resilient material. This enables the collar of the lock member to snap-fit about the second end of the connector.

According to a second aspect of the invention there is provided a feeding extension set, comprising
an enteral feeding solution supply tube,
a connector according to the first aspect of the invention, wherein the connector is configured for rotatably coupling the enteral feeding solution tube to a circular port of a gastrostomy device.

According to a third aspect of the invention there is provided a non-surgical and non-therapeutic method of delivering an enteral fluid to a patient comprising the steps of:
(i) using a connector according to the first aspect of the invention,
(ii) coupling the first end of the fluid conduit to the circular port of an indwelling gastrostomy device,
(iii) coupling the second end of the fluid conduit to an enteral feeding solution supply tube.

The method may further comprise the step of positioning the pair of hooks on a lock member provided on the connector around at least part of each flexible bridge to prevent the second free end of each arm from decoupling from the circumferential rim. This prevent inadvertent decoupling of the connector from the gastrostomy device.

A connector used to connect a fluid conduit for transporting enteral feeding solution to a port on a gastrostomy device is conventionally provided with a non-return valve. This valve ensures that the enteral feeding solution only flows in one direction, i.e., into the gastrostomy device. Back-flow is prevented. An example of a non-return valve used within gastrostomy device is a duck-bill valve. A duckbill valve is a valve, generally manufactured from rubber or synthetic elastomer, and shaped like the beak of a duck. Such a valve is commonly used in medical applications to prevent contamination due to backflow.

A problem associated with existing connectors is that a user can inadvertently squeeze the exposed flange of a duck-bill valve. This squeezing results in the opening of the valve, resulting in back-flow. This problem can be solved by locating the non-return valve entirely within the connector. This prevents inadvertent inward deflection of the flange caused by the user.

Therefore, according to a fourth aspect which is not part of the invention there is provided a gastrostomy device comprising a circular port assembly for establishing a fluid connection with a source of an enteral feeding solution, wherein the circular port assembly comprises,
a first component including:
   a first circular wall member having
   an inner diameter, and
   a circumferential rim, a portion of which extends inwardly from the first circular wall member,
a second component nested within the first component and including:
   a second circular wall member having
   an outer diameter that is less than the inner diameter of the first circular wall member, wherein a gap is provided between a surface of the inwardly extending portion of the circumferential rim of the first component and an end portion of the wall member of the second component, and
   a non-return valve located within the second component, said valve having a flange, said flange being sandwiched within the gap provided between the surface of the inwardly extending circumferential rim of the first component and the end portion of the wall member of the second component to inhibit inward deflection of the flange by the user.

In some constructions of the gastrostomy device the non-return valve is a duckbill valve.

### Brief Description of the Drawings

Constructions of the present invention will be described hereinafter, by way of example only, with reference to the accompanying drawings in which like reference signs relate to like elements and in which:
FIG. 1: Illustrates a perspective view of a first construction of a connector according to the invention for rotatably coupling an enteral feeding solution supply tube to a gastrostomy device;
FIG. 2: Illustrates a top view of the first construction of the connector of FIG. 1;
FIG. 3: Illustrates a cross-section of the first construction of the connector of FIG. 1 assembled on the circular port of the gastrostomy device;
FIG. 4: Illustrates a schematic of the first construction of the connector and the gastrostomy device prior to coupling of the connector to the gastrostomy device;
FIG. 5: Illustrates a schematic of the first construction of the connector and the gastrostomy device after coupling of the connector to the gastrostomy device;
FIGs. 6a-d: Illustrates a perspective view of a lock member attached to the first construction of the connector. The lock member is used to prevent the inadvertent decoupling of the connector from the circular port of the gastrostomy device;
FIGs. 7a-b: Illustrate a port closure member for a gastrostomy device. The port is shown in an open configuration in FIG. 7a; and a closed configuration in FIG. 7b;
FIG. 8: Illustrates a cross-sectional view of an exemplary construction of a gastrostomy device in which a duckbill valve is inaccessible to the user.

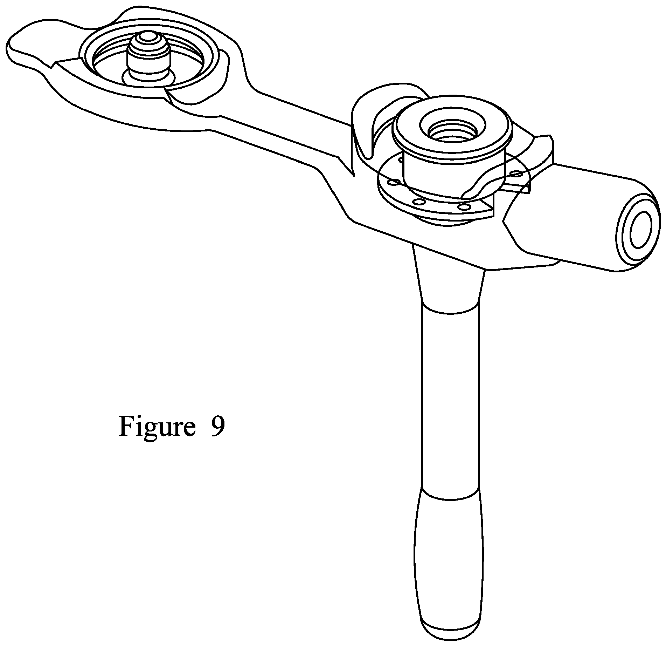

FIG. 9: Illustrates a schematic of the construction shown in FIG. 8.

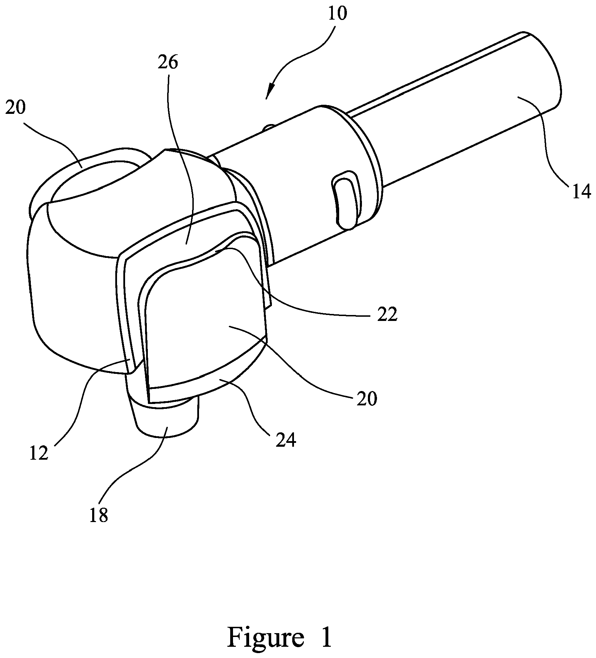

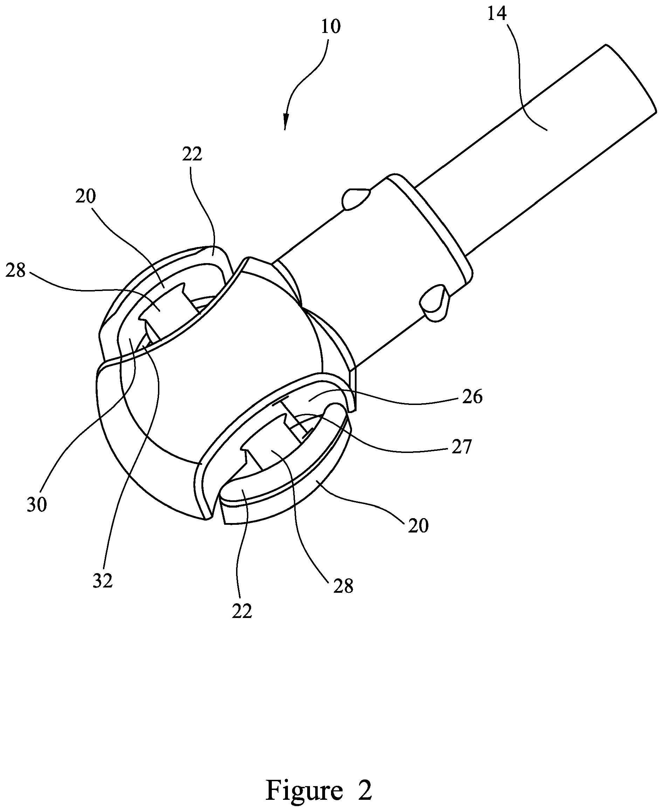

FIGs. 1 to 6 illustrate a first construction of a connector 10 according to the first aspect of the invention. The connector includes a tubular fluid conduit having a first end 12 configured for connection with an orifice 102 defined in the circular port 104 of a gastrostomy device 100. The fluid conduit also includes a second end 14 configured for connection to an enteral feeding solution supply tube (not shown). In the construction shown, the fluid conduit is generally cylindrical and has a circular cross section. The first end 12 is substantially perpendicular to the second end 14. In other constructions, the fluid conduit is straight, such that the first end 12 and the second end 14 are in-line with each other.

In the construction shown, the first end 12 of the fluid conduit has a terminally-located orifice-connecting portion 18 that is nozzle-shaped or tapered. This portion forms a tapered connection with the inner surface of the orifice 102.

The connector includes a pair of arms 20 circumferentially arranged about the first end 12 of the conduit. In the construction shown, the arms are positioned on opposing sides of the first end 12 of the conduit. Each arm includes a first free end 22 and a second free end 24.

Each arm 20 is connected to the outer wall 26 of the first end 12 of the fluid conduit via a flexible bridge 28. This flexible bridge defines a pivot about which the first and second free ends of each arm can be pivoted. A gap 27 is defined between the outer wall 26 and the inner wall 30 of each arm.

Each arm is connected such that the terminally-located orifice-connecting portion 18 extends a distance beyond the second free end 24 of each arm, thereby enabling the terminally-located orifice-connecting portion 18 to be inserted into the orifice 102.

In the construction shown, the outer wall 26 of the first end of the fluid conduit and the inner wall 30 of each arm 20 are substantially parallel, in both the coupled and decoupled configurations.

The second free end 24 of each arm includes a catch 32 that is configured to releasably engage with a circumferential rim 106 extending about the circular port 104 of the gastrostomy device 100. The catch 32 in the construction shown is located on the inner wall 30 of each arm 20, and takes the form of a ledge that extends inwardly towards the outer wall of the first end of the fluid conduit. Each ledge has a rim-contacting surface 36 that is configured to releasably engage with a ledge-contacting surface 108 on the circumferential rim 106.

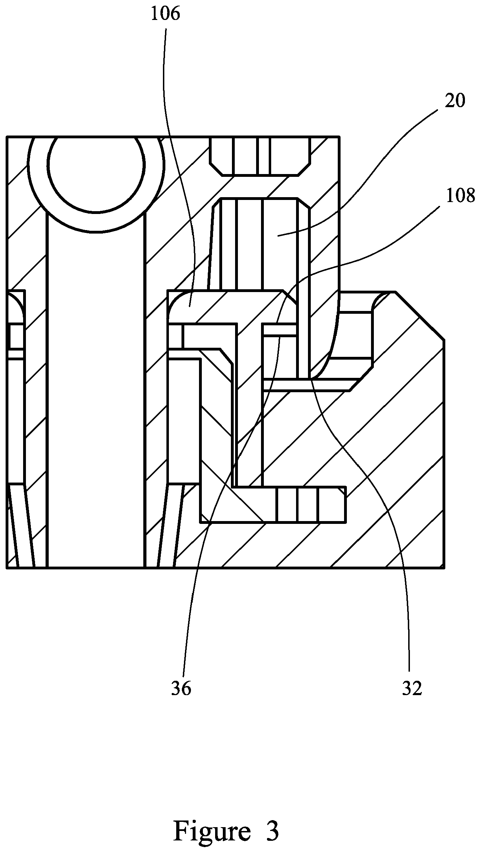

Referring now to FIG. 3, in the construction of the connector shown, the rim-contacting surface 36 and the ledge-contacting surface 108 are provided with a corresponding angled surfaces. This creates a hook effect and improves the grip of the connector against the circumferential rim.

The connector 10 may be molded as a unitary component from a plastic.

The connector 10 is couplable to and decouplable from the circular port 104 by pivoting the first and second ends of each arm at the flexible bridge 28 such that the catch 32 on the second free end 24 of each arm is radially displaced into engagement or out of engagement with the circumferential rim 106.

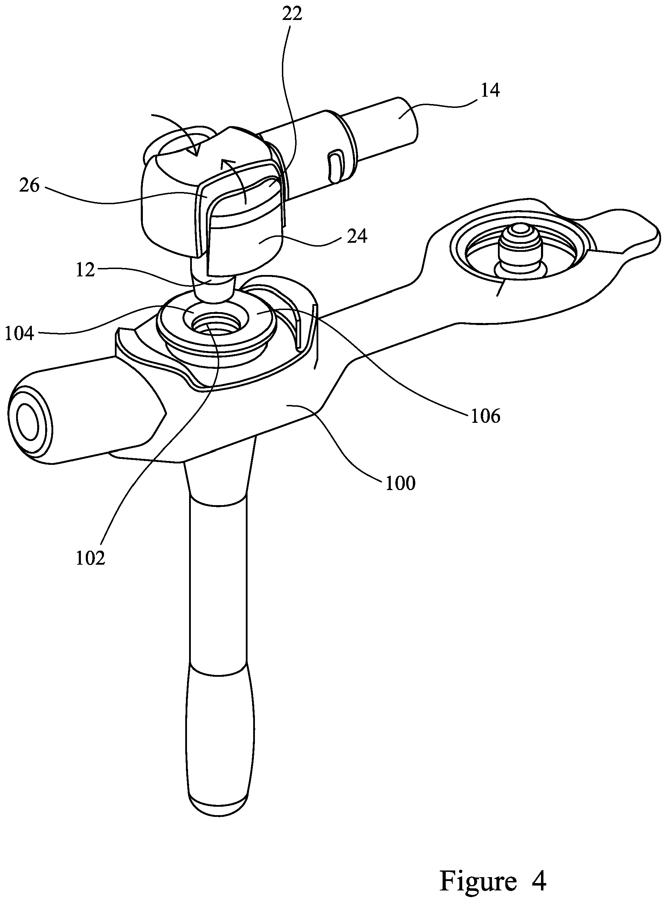

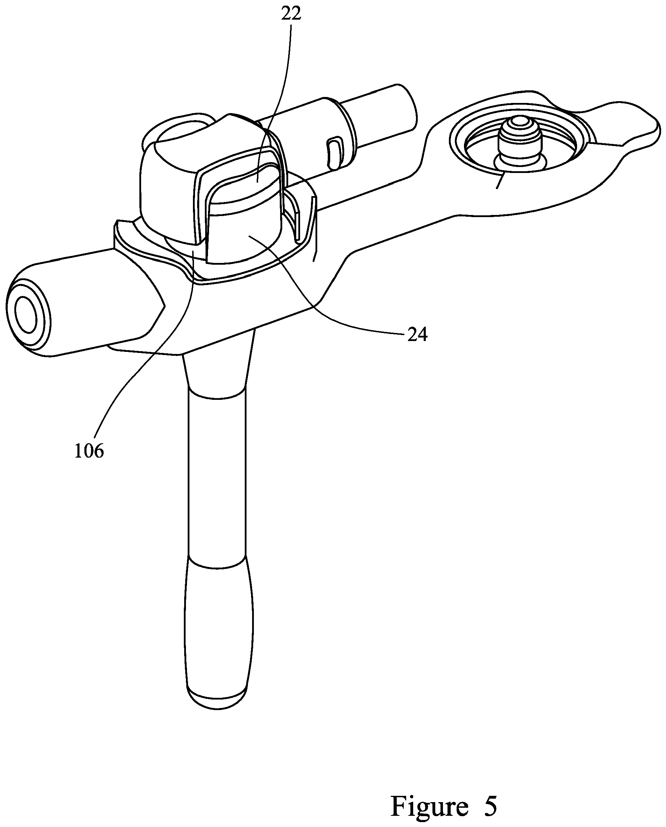

In use, in order to couple the connector 10 to the circular port 104 of the gastrostomy device, a patient or caregiver may apply an inwardly directed pressure (e.g., applied by a pinching action using the thumb and forefinger) to the outer surfaces of each arm 20 in order to move the first free end 22 of each arm inwardly towards the outer wall 26 of the first end 12 of the fluid conduit. The direction of the applied pressure is indicated by the arrows in FIG. 4. As a consequence of the inward movement of the first free end 22 of each arm, the second free end 24 of each arm is pivoted about the flexible bridge 28 in an outwardly direction away from the outer wall 26 of the first end 12 of the fluid conduit. This outward movement enables the catch 32 on the inner wall 30 of the arm to clear the circumferential rim 106 as the connector is pushed into contact with the gastrostomy device. When the user releases the applied pressure, the second free end 24 of each arm reverts to its original position. Accordingly, the rim-contacting surface 36 of the ledge engages with the ledge-contacting surface 108 on the circumferential rim 106. This is shown in FIG. 5.

Alternatively, in order to couple the connector 10 to the circular port 104 of the gastrostomy device, a patient or caregiver may simply push the second free end 24 of each arm down against the circular port 104. The angled leading edges of the catches 32 then push and flex the free end 24 outwardly such that they clip over the circumferential rim 106 as the connector is pushed into contact with the gastrostomy device.

In order to decouple the connector 10 from the circular port 104 of the gastrostomy device, the patient or caregiver performs a similar pinching actions as outlined above in relation to coupling the connector. The user applies an inwardly directed pressure (e.g., applied by a pinching action between the thumb and forefinger) to the outer surfaces of each arm 20 in order to move the first free end 22 of each arm inwardly towards the outer wall 26 of the first end 12 of the fluid conduit. As a consequence, the second free end 24 of each arm is pivoted in an outwardly direction away from the outer wall 26 of the first end 12 of the fluid conduit. This outward movement enables the catch 32 on the inner wall 30 of the arm to clear the circumferential rim 106, enabling the connector to be decoupled from the gastrostomy device.

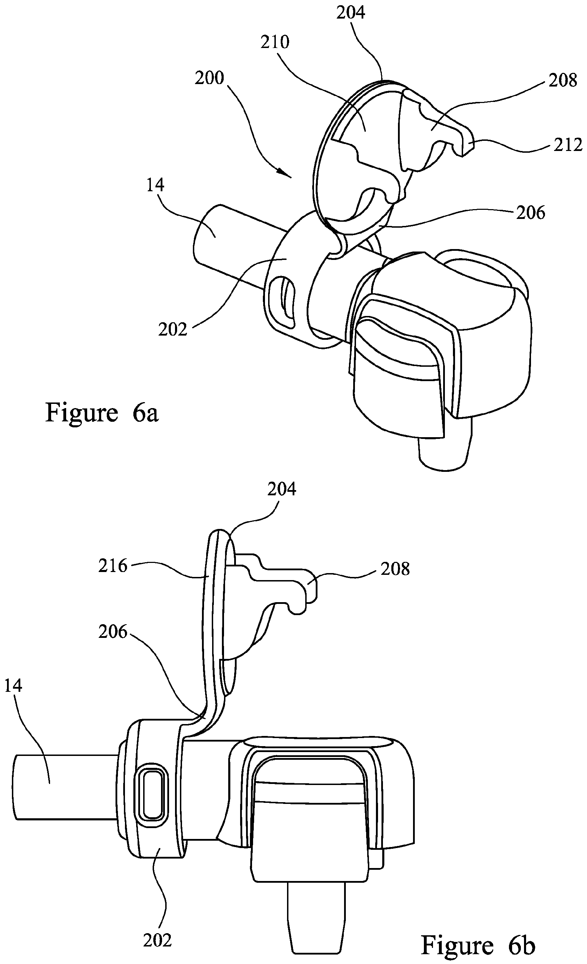

FIGs. 6a-d illustrate a perspective view of a lock member 200 attached to the first construction of the connector 10. Use of the lock member 200 prevents inadvertent decoupling of the connector from the gastrostomy device.

The lock member includes an annular collar 202 that is slidably received over the second end 14 of the tubular fluid conduit. The collar retains the lock member on the connector. The lock member also includes a generally disk-shaped cover portion 204 that is hingedly attached to the collar.

The lock member 200 includes a flexible tab portion 206 that extends between the cover portion and the collar. The flexible tab portion 206 provides a hinged connection.

A pair of spaced apart hooks 208 depend from a first surface 210 of the cover portion. Each hook 208 is dimensioned such that it is capable of being sandwiched within the gap 27 located between the between the outer wall 26 of the fluid conduit and the inner wall 30 of each arm 20 of the connector.

The curved end 212 of each hook 208 is configured to hook around at least a part of each flexible bridge 28.

As shown in FIGs. 6a-6b, the lock member 200 is molded as a unitary plastic component, with the flexible arm 206 being biased to a position in which the first surface 210 from which the hooks 208 depend is positioned substantially perpendicular to the first end 12 of the tubular fluid conduit, when the connector is not coupled to the gastrostomy device. This biasing is achieved as a result of the flexible tab portion 206 being curved.

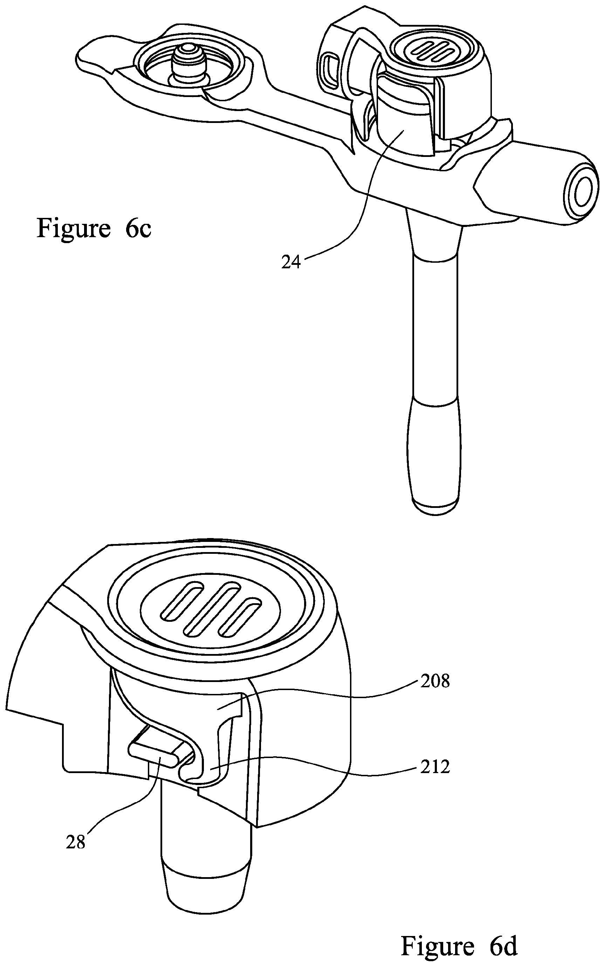

As shown in FIG. 6b, once the connector has been connected to the port of the gastrostomy device, and enteral feeding is taking place, the user pushes against the second surface 216 of the cover to rotate the cover by 90° in a clockwise direction. This causes the flexible tab portion 206 to become substantially straight and the hooks 208 are forced into the gap 27. This locked configuration is shown in FIG. 6c. In this position, the curved end 212 of each hook becomes hooked around each flexible bridge 28. As shown in FIG. 6d.

The hooks 208 act to prevent the second end 24 of each arm 20 being pivoted at the flexible bridge 28, and hence radially displaced out of engagement with the circumferential rim 106 of the circular port. Essentially, each arm is locked in the coupled configuration.

When the user wants to decouple the connector from the gastrostomy device, he/she will grip the cover and rotate it by 90° in an anti-clockwise direction, to its original position.

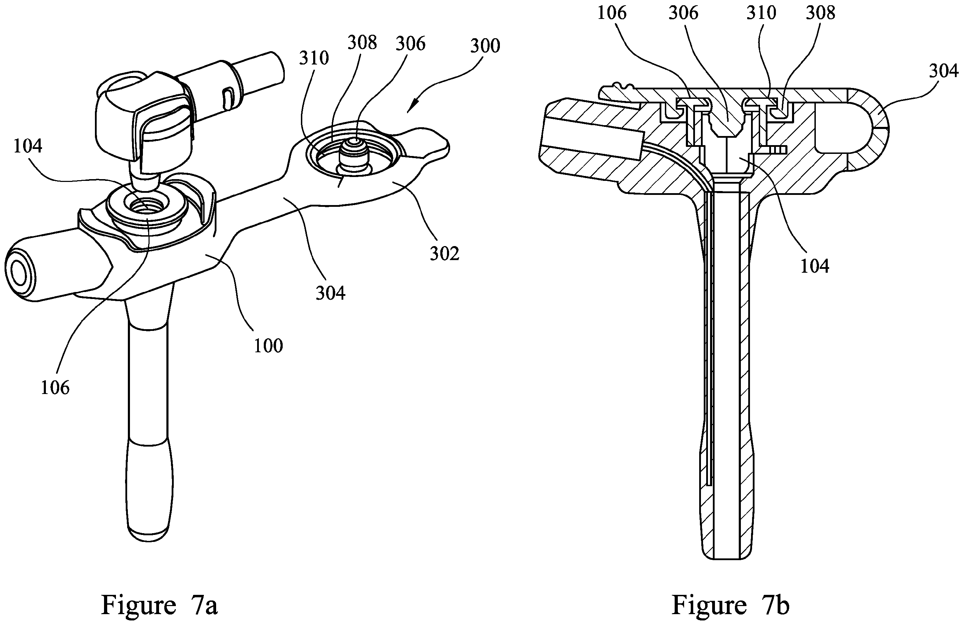

FIGs. 7a-7b illustrate a perspective view of a port closure member 300 connected to a gastrostomy device 100 according to the third aspect of the invention. FIG. 7a shows the circular port 104 open. FIG. 7b shows the circular port 104 closed by the port closure member 300. The port is closed when enteral feeding is not taking place.

The port closure member 300 comprises a cap 302 that is tethered by a flexible strip 304 to the gastrostomy device. The cap 302 includes a centrally located, generally cylindrical plug 306 that is dimensioned to be press-fitted into the circular port 104 of the device. This connection forms a fluid-tight seal. The cap also includes a ring member 308 that is radially disposed about the plug 306. The ring member 308 has a circumferential groove 310 dimensioned to receive the circumferential rim 106 provided on the circular port 104 of the gastrostomy device 100. The connection between the circular groove 310 and the circumferential rim 106 is shown in detail in the cross-sectional drawing, FIG. 7b.

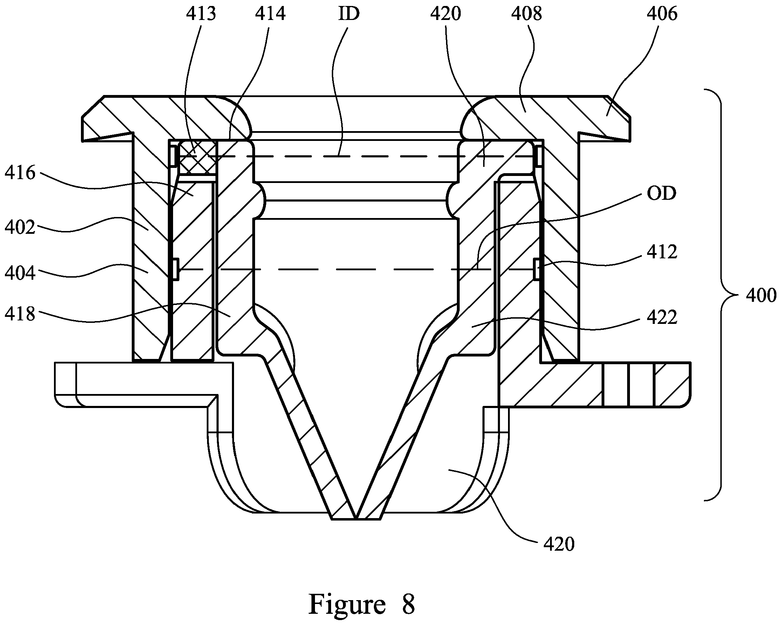

FIG. 8 illustrates a gastrostomy device which is not according to the invention.
The circular port assembly 400 of the gastrostomy device 100 consists of an interlock formed by two plastic components that are connected together. For example, the components may be ultrasonically welded together, or connected via a press-fit. A non-return duckbill valve is trapped within the assembly.

The assembly includes a first component 402 that includes a first cylindrical wall member 404 having an inner diameter (ID) and a circumferential rim 406, a portion 408 of which extends inwardly.

The assembly also includes second component 410 that includes a second cylindrical wall member 412 having an outer diameter (OD). The OD of the second cylindrical wall member 412 is less than the ID of the first cylindrical wall member 404. Accordingly, the second component is nested within the first component.

A gap 413 is provided between a surface 414 of the inwardly extending portion 408 of the circumferential rim 406 of the first component, and an end portion 416 of the second cylindrical wall member 412 of the second component.

The circular port assembly 400 also includes a non-return valve, here shown in the form of a duckbill valve 418. The duckbill valve 418 includes a flange 420 from which elastomeric lips 422 in the shape of a duckbill extend.

During assembly of the port assembly 400, the flange 420 of the duckbill valve is retained within the gap 413 provided between a surface 414 of the inwardly extending portion 408 of the circumferential rim 406 of the first component, and an end portion 416 of the second cylindrical wall member 412 of the second component. As this design locates the flange of the duckbill valve entirely within the interior of the circular port assembly, this prevents access to the flange. This minimises the risk of the user inadvertently causing the inward deflection of the flange, and the consequent opening of the valve.

## Claims

1. A connector (10) for rotatably coupling an enteral feeding solution supply tube to a circular port (104) of a gastrostomy device (100), the connector (10) comprising:
a fluid conduit having a first end (12) configured for connection with an orifice (102) defined in the circular port (104) to supply the enteral feeding solution to the gastrostomy device (100), and a second end (14) configured for connection to the enteral feeding solution supply tube,
a pair of arms (20) circumferentially arranged around an outer wall (26) of the first end (12) of the fluid conduit, each arm (20) including a first free end (22) and a second free end (24), wherein the second free end (24) includes a catch (32) configured to releasably engage with a circumferential rim (106) on the circular port (104) and
a flexible bridge (28) connecting each arm (20) to the first end (12) of the fluid conduit and about which the first and second free ends (22, 24) of each arm (20) can be pivoted,
wherein the connector (10) is couplable to and decouplable from the circular port (104) by pivoting the first and second ends (22, 24) of each arm (20) at the flexible bridge (28) such that the catch (32) on the second free end (24) of each arm (20) is radially displaced into engagement or out of engagement with the rim (106),
wherein the outer wall (26) of the first end (12) of the fluid conduit and an inner wall (30) of each arm (20) are substantially parallel,
wherein the catch (32) is a ledge located on the inner wall (30) of the arm (20) that projects inwardly towards the first end (12) of the fluid conduit, wherein the ledge has a rim-contacting surface (36) configured to releasably engage with a ledge-contacting surface (108) of the circumferential rim (106), wherein the ledge-contacting surface (108) of the rim (106) is a sloped surface and the rim-contacting surface (36) of the ledge has a complementary sloped surface and
wherein the connector (10) is moulded as a unitary component from a plastic.

2. The connector (10) according to claim 1 further comprising a lock member (200) at least a part of which is configured to be sandwiched between the outer wall (26) of the fluid conduit and the inner wall (30) of each arm (20).

3. The connector (10) according to claim 2 wherein the lock member (200) is removably attachable to the connector (10).

4. The connector (10) according to claim 2 or 3 wherein the lock member (200) is retained on the connector (10) by a collar (202) which comprises an open ring that is made of a resilient material.

5. The connector (10) according to claim 2, wherein the second end (14) of the fluid conduit is substantially perpendicular to the first end (12) of the fluid conduit, wherein the lock member (200) comprises:
a collar (202) for connecting the lock member (200) to the second end (14) of the fluid conduit, and
a cover portion (204) hingedly attached to the collar (202), wherein a pair of hooks (208) extend from the cover portion (204),
and wherein the cover portion (204) is biased such that the surface (210) from which the hooks (208) depend is substantially perpendicular to the second end (14) of the fluid conduit.

6. A feeding extension set comprising
an enteral feeding solution supply tube,
a connector (10) according to any preceding claim configured for rotatably coupling the enteral feeding solution tube to a circular port (104) of a gastrostomy device (100).

7. A non-surgical and non-therapeutic method of delivering an enteral fluid to a patient comprising the steps of:
using a connector (10) according to any of claims 1 to 5,
coupling the first end (12) of the fluid conduit to the circular port (104) of an indwelling gastrostomy device (100),
coupling the second end (14) of the fluid conduit to an enteral feeding solution supply tube.

8. The method of delivering an enteral fluid to a patient according to claim 7 when dependent on claim 5, wherein the method further comprises the step of positioning the pair of hooks (208) on the lock member (200) around at least part of each flexible bridge (28) to prevent the second free end (24) of each arm (20) from decoupling from the circumferential rim (106).

## Patentansprüche

1. Ein Verbindungsstück (10) zum drehbaren Koppeln eines Zuführschlauchs für enterale Ernährungslösung mit einem kreisförmigen Anschluss (104) einer Gastrostomievorrichtung (100), wobei das Verbindungsstück (10) Folgendes beinhaltet:
eine Fluidleitung, die ein erstes Ende (12), das zur Verbindung mit einer in dem kreisförmigen Anschluss (104) definierten Öffnung (102) konfiguriert ist, um die enterale Ernährungslösung der Gastrostomievorrichtung (100) zuzuführen, und
ein zweites Ende (14), das zur Verbindung mit dem Zuführschlauch für enterale Ernährungslösung konfiguriert ist, aufweist,
ein Paar Arme (20), die im Umfang um eine Außenwand (26) des ersten Endes (12) der Fluidleitung herum angeordnet sind, wobei jeder Arm (20) ein erstes freies Ende (22) und ein zweites freies Ende (24) aufweist, wobei das zweite freie Ende (24) eine Verriegelung (32) aufweist, die konfiguriert ist, um lösbar in einen Umfangsrand (106) an dem kreisförmigen Anschluss (104) einzugreifen, und
eine flexible Brücke (28), die jeden Arm (20) mit dem ersten Ende (12) der Fluidleitung verbindet und um die das erste und das zweite freie Ende (22, 24) jedes Arms (20) geschwenkt werden können,
wobei das Verbindungsstück (10) mit dem kreisförmigen Anschluss (104) gekoppelt und von diesem abgekoppelt werden kann, indem das erste und das zweite Ende (22, 24) jedes Arms (20) an der flexiblen Brücke (28) derart geschwenkt werden, dass die Verriegelung (32) an dem zweiten freien Ende (24) jedes Arms (20) radial in Eingriff oder außer Eingriff mit dem Rand (106) verschoben wird,
wobei die Außenwand (26) des ersten Endes (12) der Fluidleitung und eine Innenwand (30) jedes Arms (20) im Wesentlichen parallel sind,
wobei die Verriegelung (32) eine Leiste ist, die an der Innenwand (30) des Arms (20) befindlich ist und nach innen in Richtung des ersten Endes (12) der Fluidleitung vorsteht, wobei die Leiste eine Randkontaktfläche (36) aufweist, die konfiguriert ist, um lösbar mit einer Leistenkontaktfläche (108) des Umfangsrands (106) in Eingriff zu kommen, wobei die Leistenkontaktfläche (108) des Rands (106) eine geneigte Fläche ist und die Randkontaktfläche (36) der Leiste eine komplementäre geneigte Fläche aufweist, und
wobei das Verbindungsstück (10) als eine einstückige Komponente aus einem Kunststoff geformt ist.

2. Verbindungsstück (10) gemäß Anspruch 1, das ferner ein Sperrglied (200) beinhaltet, von dem mindestens ein Teil konfiguriert ist, um zwischen der Außenwand (26) der Fluidleitung und der Innenwand (30) jedes Arms (20) eingefügt zu werden.

3. Verbindungsstück (10) gemäß Anspruch 2, wobei das Sperrglied (200) abnehmbar an dem Verbindungsstück (10) angebracht werden kann.

4. Verbindungsstück (10) gemäß Anspruch 2 oder 3, wobei das Sperrglied (200) an dem Verbindungsstück (10) durch einen Kragen (202) gehalten wird, der einen offenen Ring beinhaltet, der aus einem elastischen Material hergestellt ist.

5. Verbindungsstück (10) gemäß Anspruch 2, wobei das zweite Ende (14) der Fluidleitung im Wesentlichen senkrecht zu dem ersten Ende (12) der Fluidleitung ist, wobei das Sperrglied (200) Folgendes beinhaltet:
einen Kragen (202) zum Verbinden des Sperrglieds (200) mit dem zweiten Ende (14) der Fluidleitung, und
einen Abdeckungsabschnitt (204), der scharnierartig an dem Kragen (202) angebracht ist, wobei sich ein Paar Haken (208) von dem Abdeckungsabschnitt (204) erstrecken,
und wobei der Abdeckungsabschnitt (204) derart vorgespannt ist, dass die Fläche (210), von der die Haken (208) herabhängen, im Wesentlichen senkrecht zu dem zweiten Ende (14) der Fluidleitung ist.

6. Ein Ernährungs-Verlängerungsset, das Folgendes beinhaltet:
einen Zuführschlauch für enterale Ernährungslösung,
ein Verbindungsstück (10) gemäß einem der vorhergehenden Ansprüche, das konfiguriert ist, um den Schlauch für enterale Ernährungslösung drehbar mit einem kreisförmigen Anschluss (104) einer Gastrostomievorrichtung (100) zu koppeln.

7. Ein nicht chirurgisches und nicht therapeutisches Verfahren zum Abgeben eines enteralen Fluids an einen Patienten, das folgende Schritte beinhaltet:
Verwenden eines Verbindungsstücks (10) gemäß einem der Ansprüche 1 bis 5,
Koppeln des ersten Endes (12) der Fluidleitung mit dem kreisförmigen Anschluss (104) einer Dauergastrostomievorrichtung (100),
Koppeln des zweiten Endes (14) der Fluidleitung mit einem Zuführschlauch für enterale Ernährungslösung.

8. Verfahren zum Abgeben eines enteralen Fluids an einen Patienten gemäß Anspruch 7, wenn von Anspruch 5 abhängig, wobei das Verfahren ferner den Schritt des Positionierens des Paares Haken (208) auf dem Sperrglied (200) um mindestens einen Teil jeder flexiblen Brücke (28) beinhaltet, um zu verhindern, dass sich das zweite freie Ende (24) jedes Arms (20) von dem Umfangsrand (106) abkoppelt.

## Revendications

1. Un connecteur (10) destiné à raccorder de façon à ce qu'elle puisse tourner une tubulure d'apport de solution d'alimentation entérale à une lumière circulaire (104) d'un dispositif de gastrostomie (100), le connecteur (10) comprenant :
un conduit de fluide ayant une première extrémité (12) configurée pour la connexion avec un orifice (102) défini dans la lumière circulaire (104) afin d'apporter la solution d'alimentation entérale au dispositif de gastrostomie (100),
et une deuxième extrémité (14) configurée pour la connexion à la tubulure d'apport de solution d'alimentation entérale,
une paire de bras (20) agencés de façon circonférentielle autour d'une paroi externe (26) de la première extrémité (12) du conduit de fluide, chaque bras (20) incluant une première extrémité libre (22) et une deuxième extrémité libre (24), la deuxième extrémité libre (24) incluant un cran (32) configuré pour se mettre en engagement libérable avec un rebord circonférentiel (106) sur la lumière circulaire (104) et
un pont souple (28) connectant chaque bras (20) à la première extrémité (12) du conduit de fluide et autour duquel la première et la deuxième extrémité libre (22, 24) de chaque bras (20) peuvent être faites pivoter,
dans lequel le connecteur (10) peut être raccordé à et détaché de la lumière circulaire (104) en faisant pivoter la première et la deuxième extrémité (22, 24) de chaque bras (20) au niveau du pont souple (28) de sorte que le cran (32) sur la deuxième extrémité libre (24) de chaque bras (20) est déplacé radialement pour venir en engagement avec ou pour se désengager d'avec le rebord (106),
dans lequel la paroi externe (26) de la première extrémité (12) du conduit de fluide et une paroi interne (30) de chaque bras (20) sont substantiellement parallèles,
dans lequel le cran (32) est une corniche se trouvant sur la paroi interne (30) du bras (20) qui fait saillie vers l'intérieur en direction de la première extrémité (12) du conduit de fluide, la corniche ayant une surface (36) en contact avec le rebord configurée pour se mettre en engagement libérable avec une surface (108) en contact avec la corniche du rebord circonférentiel (106), la surface en contact avec la corniche (108) du rebord (106) étant une surface inclinée et la surface en contact avec le rebord (36) de la corniche ayant une surface inclinée complémentaire et
dans lequel le connecteur (10) est moulé en tant que composant unitaire à partir d'un plastique.

2. Le connecteur (10) selon la revendication 1 comprenant en sus un élément de verrouillage (200) dont au moins une partie est configurée pour être prise en sandwich entre la paroi externe (26) du conduit de fluide et la paroi interne (30) de chaque bras (20).

3. Le connecteur (10) selon la revendication 2 dans lequel l'élément de verrouillage (200) peut être attaché de façon amovible au connecteur (10).

4. Le connecteur (10) selon la revendication 2 ou la revendication 3 dans lequel l'élément de verrouillage (200) est retenu sur le connecteur (10) par un collier (202) qui comprend une bague ouverte qui est faite en un matériau résilient.

5. Le connecteur (10) selon la revendication 2, dans lequel la deuxième extrémité (14) du conduit de fluide est substantiellement perpendiculaire à la première extrémité (12) du conduit de fluide, dans lequel l'élément de verrouillage (200) comprend :
un collier (202) pour connecter l'élément de verrouillage (200) à la deuxième extrémité (14) du conduit de fluide, et
une portion formant couvercle (204) attachée de façon articulée au collier (202), une paire de crochets (208) s'étendant à partir de la portion formant couvercle (204),
et dans lequel la portion formant couvercle (204) est sollicitée de telle sorte que la surface (210) dont dépendent les crochets (208) est substantiellement perpendiculaire à la deuxième extrémité (14) du conduit de fluide.

6. Un ensemble-rallonge d'alimentation comprenant
une tubulure d'apport de solution d'alimentation entérale,
un connecteur (10) selon n'importe quelle revendication précédente configuré pour raccorder de façon à ce qu'elle puisse tourner la tubulure de solution d'alimentation entérale à une lumière circulaire (104) d'un dispositif de gastrostomie (100).

7. Un procédé non chirurgical et non thérapeutique d'apport d'un fluide entéral à un patient comprenant les étapes consistant :
à utiliser un connecteur (10) selon n'importe lesquelles des revendications 1 à 5,
à raccorder la première extrémité (12) du conduit de fluide à la lumière circulaire (104) d'un dispositif de gastrostomie à demeure (100),
à raccorder la deuxième extrémité (14) du conduit de fluide à une tubulure d'apport de solution d'alimentation entérale.

8. Le procédé d'apport d'un fluide entéral à un patient selon la revendication 7 lorsqu'elle dépend de la revendication 5, le procédé comprenant en sus l'étape de mise en position de la paire de crochets (208) sur l'élément de verrouillage (200) autour d'au moins une partie de chaque pont souple (28) afin d'empêcher que la deuxième extrémité libre (24) de chaque bras (20) se détache du rebord circonférentiel (106).
